# EUROPEAN PATENT SPECIFICATION

(11) **EP 1 828 380 B1**
(45) Date of publication and mention of the grant of the patent: **15.12.2010**
(21) Application number: 05854547.6
(22) Date of filing: 16.12.2005
(51) Int. Cl.: C12N 9/42, C11D 3/386, D06M 16/00, D21C 5/00, A23K 1/165, C12N 15/55

(54) **NEUTRAL CELLULASE CATALYTIC CORE AND METHOD OF PRODUCING SAME**
NEUTRALER CELLULASEKATALYSATORKERN UND HERSTELLUNGSVERFAHREN DAFÜR
NOYAU CATALYTIQUE DE CELLULASE NEUTRE ET PROCEDE D'ELABORATION

(30) Priority: 23.12.2004 US 638953 P
(43) Date of publication of application: 05.09.2007
(73) Proprietor: GENENCOR INTERNATIONAL, INC., Palo Alto, California 94304 (US)
(72) Inventor: BAO, Kai, GENENCOR INTERNATIONAL, INC., Palo Alto, CA 94304 (US); WANG, Huaming, GENENCOR INTERNATIONAL, INC., Palo Alto, US 94304 (US)
(74) Representative: Kiddle, Simon John
(86) International application number: PCT/US2005/045859
(87) International publication number: WO 2006/071598

(56) References cited:
- EP-A- 1 344 820
- EP-A- 1 408 108
- WO-A-00/09707
- WO-A-97/27363
- WO-A-98/04663
- WO-A-03/012109
- WO-A-03/021033
- CA-A1- 2 198 828
- US-A- 5 712 142
- DATABASE RCSB PDB Acc.no. 1AO4_A 27 March 2003 (2003-03-27), XP002380370

## Description

### FIELD OF THE INVENTION

This invention relates to a process for producing high levels of novel truncated cellulase proteins in a host cell, to host cell transformants produced by genetic engineering techniques; and to novel cellulase proteins produced by such transformants. The novel cellulase proteins are novel cellulase catalytic cores. The treatment of cellulose containing fabrics with cellulase core domains of the invention are disclosed as offering specific advantages of reduced redeposition of dye and increased abrasion.

### BACKGROUND OF THE INVENTION

Cellulases are enzymes that hydrolyze the β-D-glucosidic linkages in celluloses. Cellulolytic enzymes have been traditionally divided into three major classes: endoglucanases, exoglucanases or cellobiohydrolases and β-glucosidases (Knowles, J. et al., TIBTECH 5:255-261 (1987)). Cellulases are known to be produced by a large number of bacteria, yeasts and fungi.

Primary among the applications that have been developed for the use of cellulolytic enzymes are those involving degrading (wood) cellulose pulp into sugars for (bio)ethanol production, textile treatments like "stone washing" and "biopolishing," and in detergent compositions. Cellulases are also known to be useful in detergent compositions for removing dirt, *i*.*e*., cleaning. For example, Great Britain Application Nos. 2,075,028, 2,095,275 and 2,094,826 illustrate improved cleaning performance with detergents that have incorporated cellulase. Additionally, Great Britain Application No. 1,358,599 illustrates the use of cellulase in detergents to reduce the harsh feel of cotton-containing fabrics.

Another useful feature of cellulases in the treatment of textiles is their ability to recondition used fabrics by making their colors more vibrant. For example, repeated washing of cotton containing fabrics results in a greyish cast to the fabric. This is believed to be due to disrupted and disordered fibrils, sometimes called "pills," caused by mechanical action. This greyish cast is particularly noticeable on colored fabrics. As a consequence, the ability of cellulase to remove the disordered top layer of the fiber and thus improve the overall appearance of the fabric has been found to be of value. Because detergents, being a primary application of cellulase, operate generally under alkaline conditions there is a strong demand for cellulases that exhibit high activity at pH 7-10. Well characterized fungal cellulases, such as those from *Humicola insolens* and *Trichoderma reesei,* perform adequately at neutral to low alkaline pH. A number of enzymes demonstrating cellulase activity at high alkaline pH have been isolated from *Bacillus* and other prokaryotes, *see e.g.,* PCT Publication Nos. WO 96/34092 and WO 96/34108. Thus, both fungal and bacterial cellulases have been investigated thoroughly. However, a third group of cellulases, those isolated from *Actinomycetes,* have attracted only meager attention. Wilson, et al., Critical Reviews in Biotechnology, 12:45-63 (1992), have studied cellulases produced by *Thermomonospora fusca, Thermonomospora curvata* and *Microbispora bispora* and have shown that many of these cellulases exhibit broad pH profiles and good temperature stability. Similarly, Nakai, et al., Agric. Biol. Chem., 51: 3061-3065 (1987) and Nakai, et al., Gene, 65:229-238 (1988) have demonstrated the alkalitolerant cellulase casA from *Streptomyces* strain KSM-9. This cellulase possesses an alkaline pH optimum and excellent temperature stability.

Despite knowledge in the art related to many cellulase compositions having desirable properties, including some examples from *Actinomycetes,* there is a continued need for new cellulases having a varying spectrum of characteristics useful as, for example, textile treatments, components of detergent compositions, pulp and paper treatments, animal feed supplements, processing aids for baking, and biomass converters. Applicants have discovered cellulases which possess such a complement of characteristics and which are useful in such known applications of cellulases.

### BRIEF SUMMARY OF THE INVENTION

The present invention relates to a cellulase catalytic core domain, constructs, vectors and host cells comprising one or more of the promoter sequences operably linked to a heterologous coding sequence.

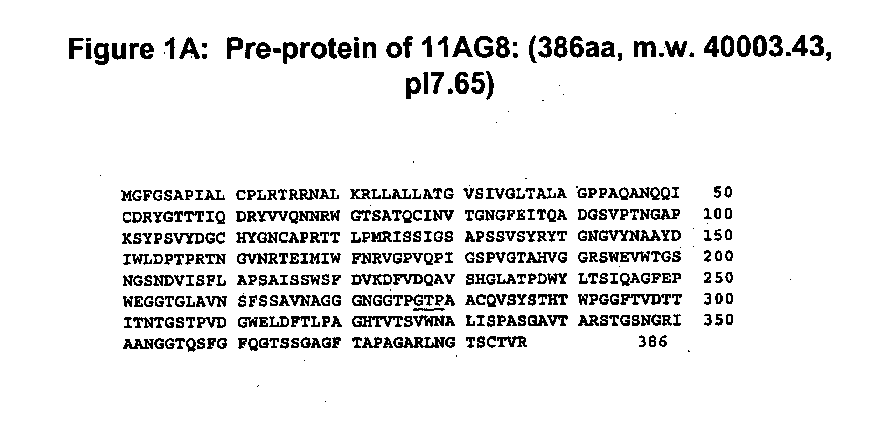

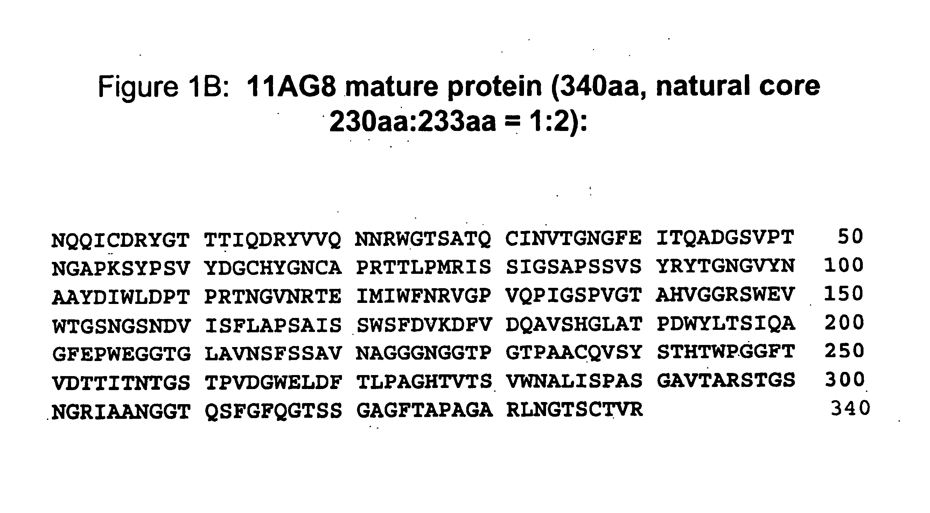

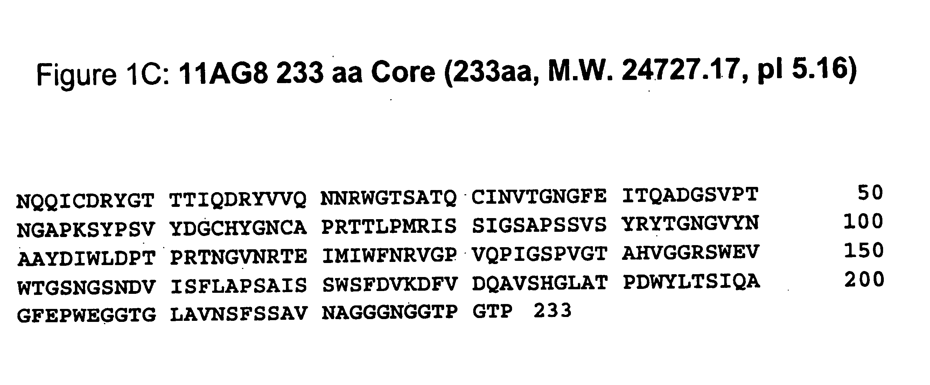

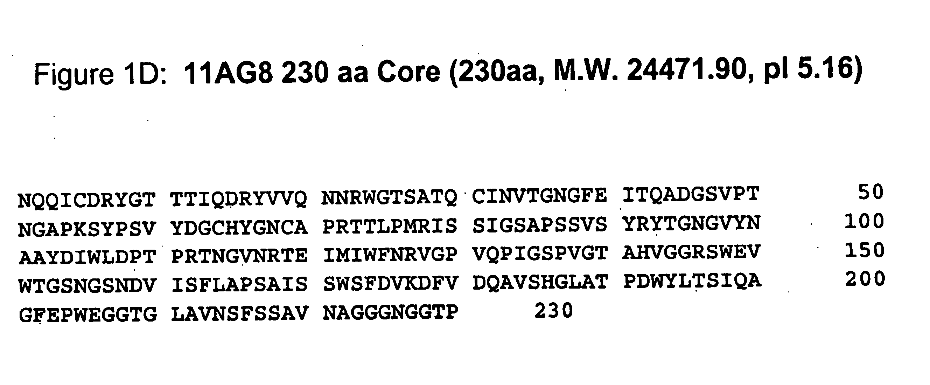

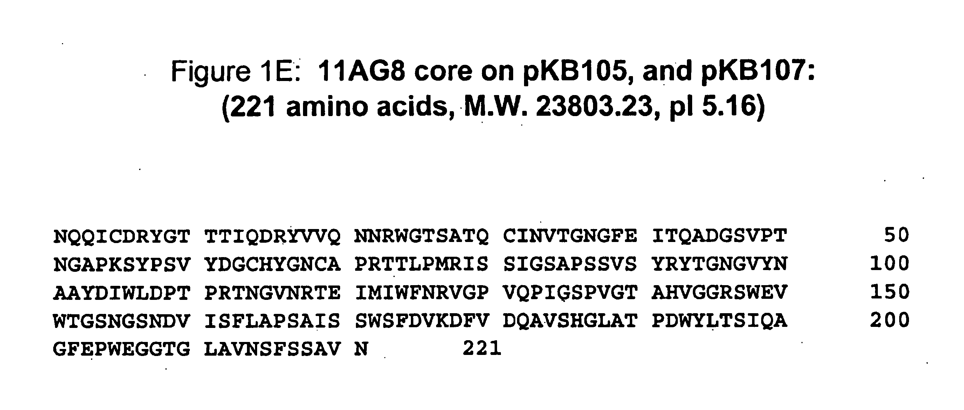

Accordingly, in one aspect, the present invention provides an isolated cellulase having an amino acid sequence consisting of the amino acid sequence as set out in Figure 1E. This cellulase has excellent properties for use in detergents, treating textiles, as a feed supplement and in pulp and paper manufacturing, as disclosed further herein.

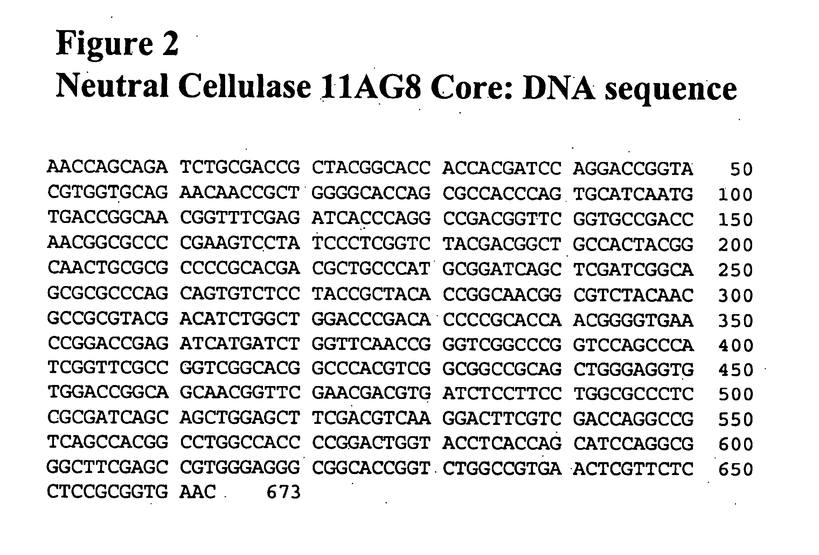

In a further aspect, the present invention provides a DNA sequence encoding the cellulase which has the nucleic acid sequence as set out in Figure 2. In further aspects, the present invention provides expression comprising the DNA set out in Figure 2, such as the vector is pKB107, and host cells that have been transformed with a vector comprising the DNA encoding the amino acid sequence presented in Figure 2. In one embodiment, when the host cell is a *Streptomyces sp*., the host cell may optionally have the cyc2 gene (geosmin pathway gene) deleted.

The present invention also relates to methods and uses of the claimed cellulases. The methods can be used for the treatment of cellulose containing fabrics with cellulase core domains as defined in claim 1. The methods and uses provide the specific advantages of reduced redeposition of dye, increased abrasion, improved fabric hand (i.e., softening), smooth fabric surface, pill removal and/or pill prevention.

In other aspects, the methods and uses of the claimed cellulases are for enhancing the digestibility of animal feed, in detergents, in the treatment of pulp and paper and in the production of starch and treatment of by-products thereof.

It should be understood, however, that the detailed description and specific examples, while indicating preferred embodiments of the invention, are given by way of illustration only, since various changes and modifications within the scope and spirit of the invention will become apparent to one skilled in the art from this detailed description.

### BRIEF DESCRIPTION OF THE DRAWINGS

Figure 1 illustrates the various cellulase proteins. Figure 1A is the pre-protein.
Figure 1B is the mature 11 AG8 cellulase protein. Figure 1C is 233 amino acid 11 AG8cellulase protein without the CBD. Figure 1D is 230 amino acid 11AG8 cellulase protein 15 without the CBD. Figure 1E is 221 amino acid 11 AG8 cellulase catalytic core, i.e., without the CBD or linker. The signal sequence is in pink. The linker is 12 amino acids in length with 9 residues in brown and 3 in red. The three amino acids that are in the 233 amino acid protein in red (and underlined in Fig. 1A only). The CBD is in blue.
Figure 2 is a DNA sequence encoding the 221 amino acid catalytic core of the 11AG8 cellulase.
Figure 3 shows the pSEGCT11 AG8 vector which includes:
   a glucose isomerase promoter derived from *Actinoplanes missouriensis,*
   a signal sequence of S. *lividans* cellulase, celA,
   a polynucleotide encoding a cellulase 11AG8 gene from an *Actinomyces* species,
   a cellulase 11 AG3 terminator sequence.
Figure 4 shows the pSEA4CT-11AG8 vector which includes:
   a A4 promoter derived from *Aspergillus niger,*
   a signal sequence of S. *lividans* cellulase, celA,
   a polynucleotide encoding a cellulase 11 AG8 gene from an *Actinomyces* species,
   a cellulase 11 AG3 terminator sequence.

   The GI promoter of pSEGCT11 AG8 has been replaced with the *Aspergillus niger* A4 promoter.

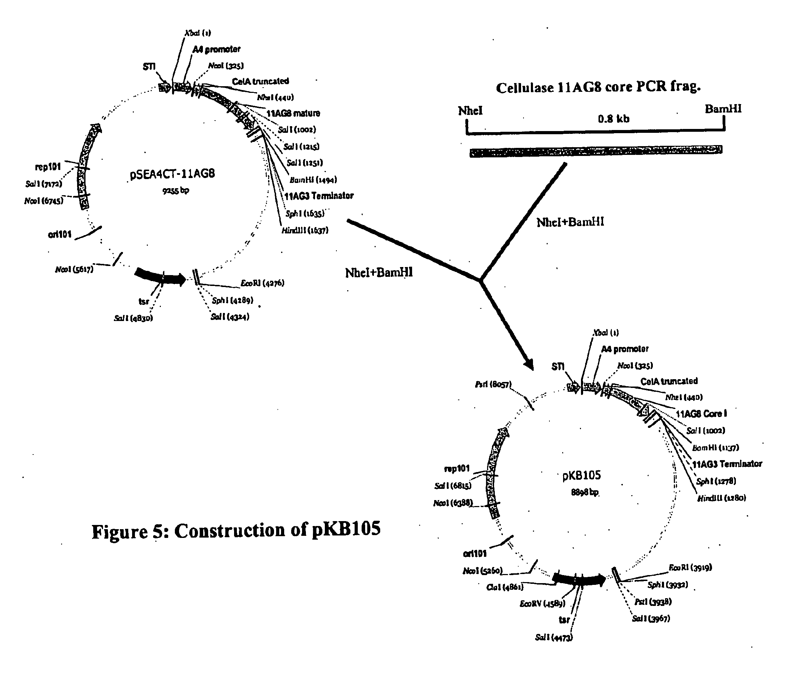

Figure 5 shows the construction strategy for pKB105.

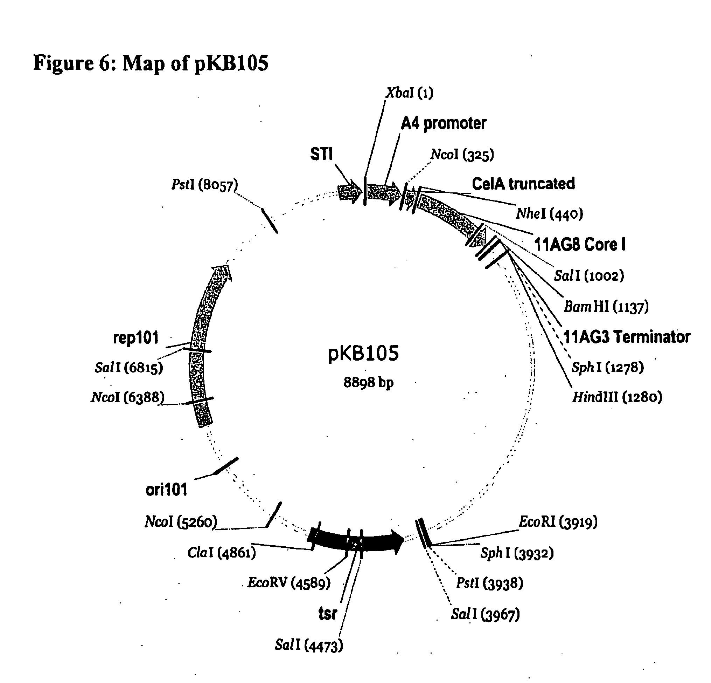

Figure 6 is the pKB105 vector which includes:
   a A4 promoter derived from *Aspergillus niger,*
   a signal sequence of *S. lividans* cellulase, celA,
   a polynucleotide encoding the 221 a.a. catalytic core from the 11AG8 cellulase from an *Actinomyces* species,
   a cellulase 11AG3 terminator sequence.

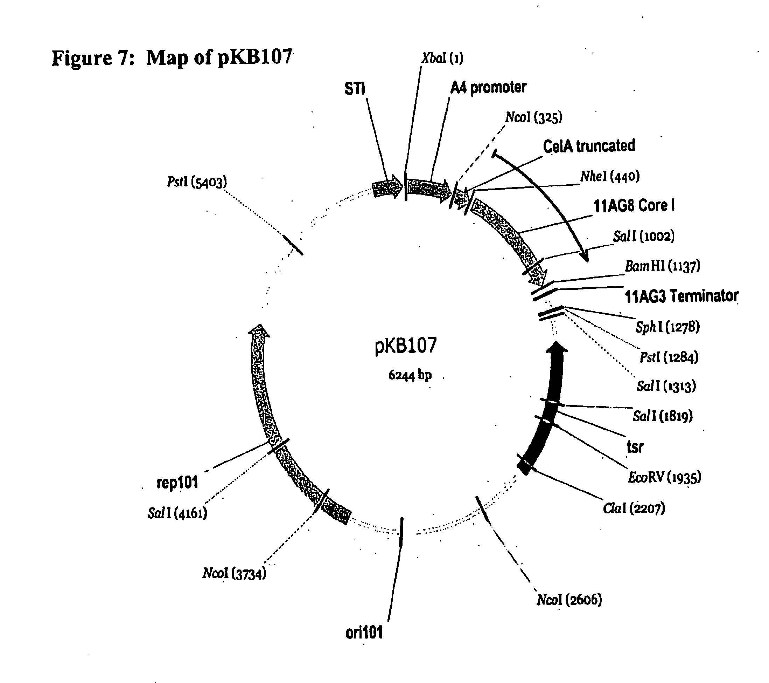

Figure 7 shows the pKB107 vector which is pKB105 with the E. coli sequences having been removed.

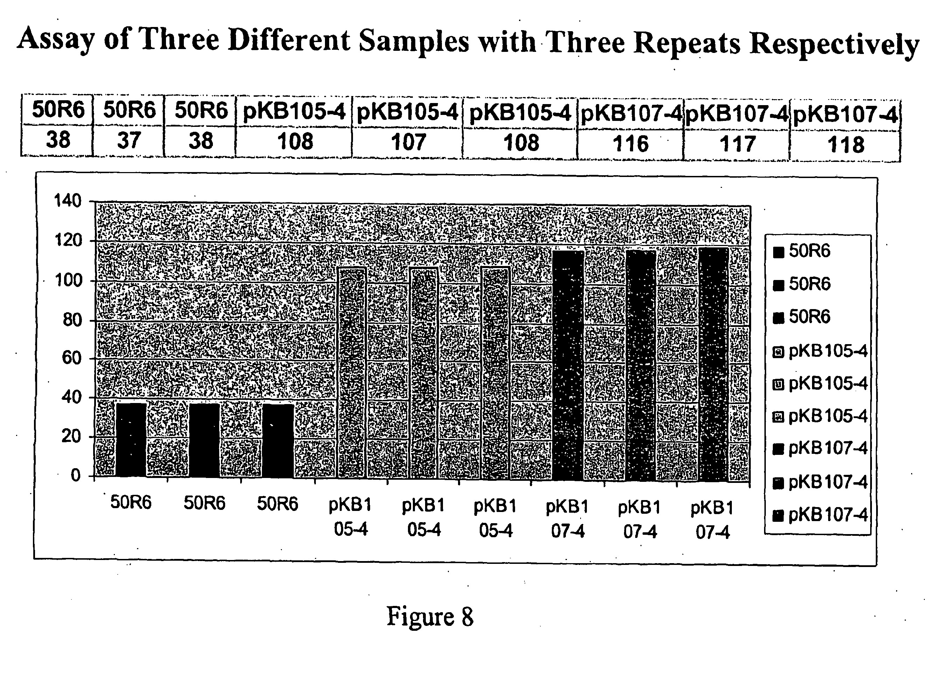

Figure 8 shows the activity of various cellulase samples in the NPC assay described herein. 50R6 is the protein sample from the commercial production strain for IndiAge^{®} Neutra L. pKB105 and pKB107 are protein samples expressed from the respective vectors. The catalytic core demonstrates a higher activity than the commercial product.

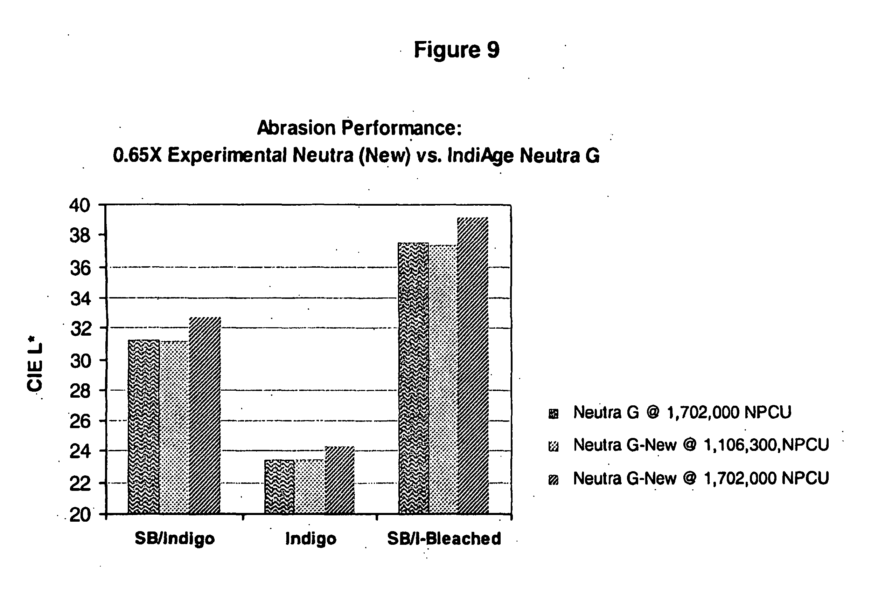

Figure 9 is a graph showing the abrasion performance of the new Neutra G (compared with the performance of the commercial product, IndiAge^{®} Neutra G. The new Neutra G dosed at 65% NPC activity exhibited very close abrasion performance of IndiAge^{®} Neutra G at 100% activity regardless of the denim type. The new Neutra G showed significantly higher abrasion performance when it was dosed at same activity of IndiAge^{®} Neutra G.

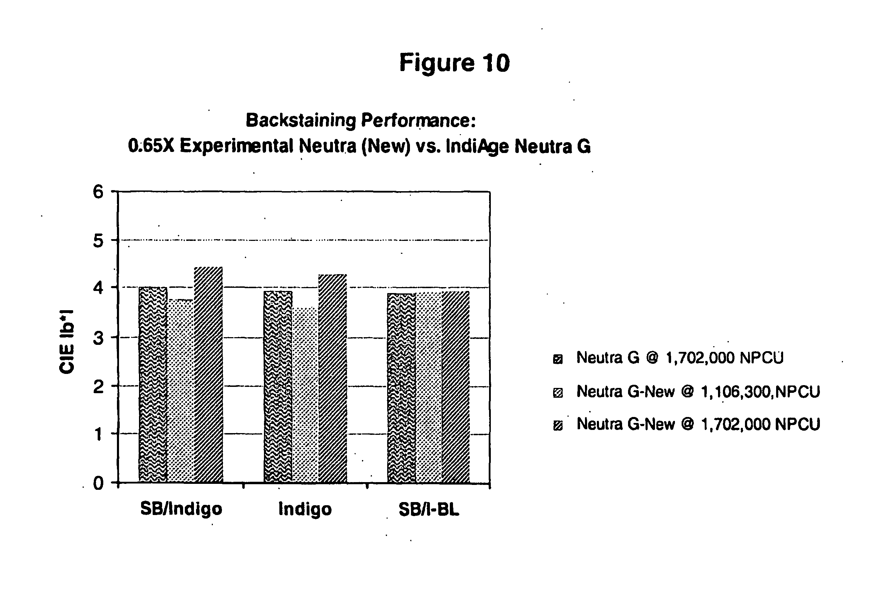

Figure 10 is a graph showing the backstaining performance the new Neutra G compared with the performance of the commercial product, IndiAge^{®} Neutra G. No significant backstaining performances were observed between the new Neutra G and current commercial IndiAge^{®} Neutra G under similar abrasion level.

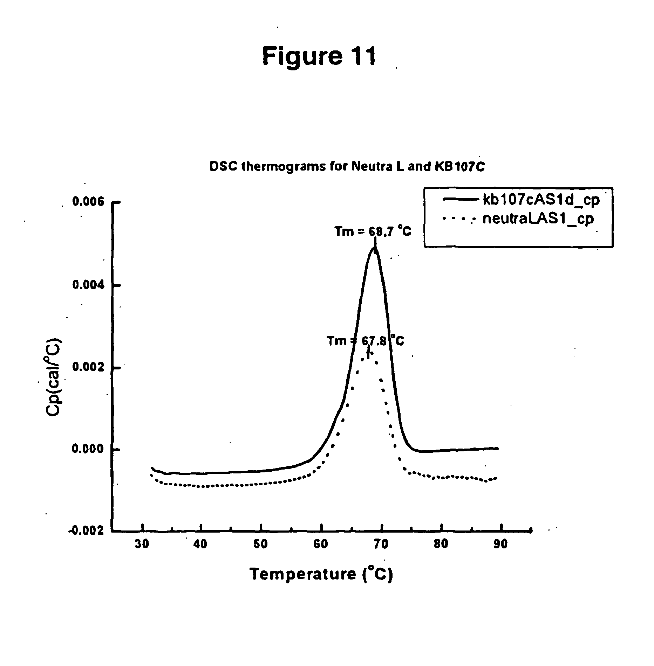

Figure 11 shows the DSC thermograms for IndiAge^{®} Neutra L and KB107C. The KB107C molecule has a melting point (Tm) of 68.7°C relative to 67.87°C for IndiAge^{®} Neutra L indicating that KB107C is more stable.

### DETAILED DESCRIPTION

The invention will now be described in detail by way of reference only using the following definitions and examples.

Unless defined otherwise herein, all technical and scientific terms used herein have the same meaning as commonly understood by one of ordinary skill in the art to which this invention belongs. Singleton, et al., DICTIONARY OF MICROBIOLOGY AND MOLECULAR BIOLOGY, 2D ED., John Wiley and Sons, New York (1994), and Hale & Marham, THE HARPER COLLINS DICTIONARY OF BIOLOGY, Harper Perennial, NY (1991) provide one of skill with a general dictionary of many of the terms used in this invention. Although any methods and materials similar or equivalent to those described herein can be used in the practice or testing of the present invention, the preferred methods and materials are described. Numeric ranges are inclusive of the numbers defining the range. Unless otherwise indicated, nucleic acids are written left to right in 5' to 3' orientation; amino acid sequences are written left to right in amino to carboxyl orientation, respectively. Practitioners are particularly directed to Sambrook *et al*., 1989, and Ausubel FM *et al.,* 1993, for definitions and terms of the art. It is to be understood that this invention is not limited to the particular methodology, protocols, and reagents described, as these may vary.

The headings provided herein are not limitations of the various aspects or embodiments of the invention which can be had by reference to the specification as a whole. Accordingly, the terms defined immediately below are more fully defined by reference to the specification as a whole.

### Definitions

The term "nucleic acid molecule" includes RNA, DNA and cDNA molecules. It will be understood that, as a result of the degeneracy of the genetic code, a multitude of nucleotide sequences encoding a given protein such as a cellulase may be produced. The present invention contemplates every possible variant nucleotide sequence, encoding the presently claimed cellulase, all of which are possible given the degeneracy of the genetic code.

The term "amino acid" includes reference to an amino acid that is incorporated into a protein, polypeptide, or peptide (collectively "polypeptide"). The amino acid can be a naturally occurring amino acid or, unless otherwise limited, can encompass known analogs of natural amino acids that function in a similar manner as naturally occurring amino acids.

"Derivative" is intended to indicate a peptide or protein that is derived from a native protein by addition of one or more amino acids to either or both the C- and N-terminal end of the native protein, substitution of one or more amino acids at one or a number of different sites in the native amino acid sequence, deletion of one or more amino acids at either or both ends of the native protein or at one or more sites in the amino acid sequence, or insertion of one or more amino acids at one or more sites in the native amino acid sequence. The preparation of an enzyme derivative is preferably achieved by modifying the DNA sequence that encodes the native protein, transformation of that DNA sequence into a suitable host, and expression of the modified DNA sequence to form the derivative enzyme. Alternative means of preparing derivatives are well known in the art, and include, *e.g*., proteolytic cleavage of native proteins or their derivatives. The derivatives of the cellulases of this invention include peptides comprising altered amino acid sequences in comparison with a precursor enzyme amino acid sequence (*e.g*., a wild type or native state enzyme according to the present invention) that retain a characteristic enzymatic nature of the precursor enzyme but which have altered properties in some specific aspect. For example, an altered cellulase may have an increased pH optimum or increased temperature resistance but will retain its characteristic cellulolytic activity. Additionally, as used herein the term "derived from" with respect to expression vector components means a sequence that is identical to a reference sequence, is a naturally occurring or engineered variant, or chemically synthesized copy or variant thereof, which preserves the desired function. Thus, reference to a promoter sequence derived from an *Actinoplanes* glucose isomerase gene would include a functional promoter sequence of the glucose isomerase gene from *A. missouriensis,* functional promoter sequences from protein-engineered derivatives, such as GIT and the like. (See European Patent Application 351029). Also, the promoter(s) described in co-pending application USSN 10/992,149 find use herein.

"Conservative substitutions" of a particular amino acid sequence refers to amino acid substitutions of those amino acids that are not critical for functional activity or substitution of amino acids with other amino acids having similar properties (*e.g*., acidic, basic, positively or negatively charged, polar or non-polar, etc.) such that the substitutions of even critical amino acids do not substantially alter activity. Conservative substitution tables providing functionally similar amino acids are well known in the art.

The term "promoter" is defined herein as a DNA sequence that binds RNA polymerase and directs the polymerase to the correct downstream transcription start site of a coding sequence of interest resulting in transcription. The term "promoter" is also understood to include the 5'non-coding region (between the promoter and translation start) for translation after transcription into mRNA and cis-acting transcription control elements, such as enhancers. The promoter will be effective in *Streptomyces* to express a coding region of interest.

A nucleic acid is "operably linked" when it is placed into a functional relationship with another nucleic acid sequence. For example, DNA encoding a secretory leader, i.e., a signal peptide, is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

As used herein, the term "gene" means the segment of DNA involved in producing a polypeptide chain, that may or may not include regions preceding and following the coding region, e.g. 5' untranslated (5' UTR) or leader" sequences and 3' UTR or "trailer" sequences, as well as intervening sequences (introns) between individual coding segments (exons).

The term "heterologous" when used with reference to portions of a nucleic acid indicates that the nucleic acid comprises two or more subsequences that are not normally found in the same relationship to each other in nature. For instance, the nucleic acid is typically recombinantly produced, having two or more sequences, e.g., from unrelated genes arranged to make a new functional nucleic acid, e.g., a promoter from one source and a coding region from another source. Similarly, a heterologous protein will often refer to two or more subsequences that are not found in the same relationship to each other in nature (e.g., a fusion protein).

The terms "isolated" or "purified" as used herein refer to a nucleic acid or amino acid that is removed from at least one component with which it is naturally associated.

In the present context, the term "substantially pure polypeptide" means a polypeptide preparation which contains at the most 10% by weight of other polypeptide material with which it is natively associated (lower percentages of other polypeptide material are preferred, e.g. at the most 8% by weight, at the most 6% by weight, at the most 5% by weight, at the most 4% at the most 3% by weight, at the most 2% by weight, at the most 1% by weight, and at the most 1/2% by weight). Thus, it is preferred that the substantially pure polypeptide is at least 92% pure, i.e. that the polypeptide constitutes at least 92% by weight of the total polypeptide material present in the preparation, and higher percentages are preferred such as at least 94% pure, at least 95% pure, at least 96% pure, at least 96% pure, at least 97% pure, at least 98% pure, at least 99%, and at the most 99.5% pure. The polypeptides disclosed herein are preferably in a substantially pure form. In particular, it is preferred that the polypeptides disclosed herein are in "essentially pure form", i.e. that the polypeptide preparation is essentially free of other polypeptide material with which it is natively associated. This can be accomplished, for example, by preparing the polypeptide by means of well-known recombinant methods. Herein, the term "substantially pure polypeptide" is synonymous with the terms "isolated polypeptide" and "polypeptide in isolated form".

The term "recombinant" when used with reference, e.g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all.

The term "secretory signal sequence" denotes a DNA sequence that encodes a polypeptide (a "secretory peptide") that, as a component of a larger polypeptide, directs the larger polypeptide through a secretory pathway of a cell in which it is synthesized. The larger peptide is commonly cleaved to remove the secretory peptide during transit through the secretory pathway.

A "vector" refers to a polynucleotide sequence designed to introduce nucleic acids into one or more cell types. Vectors include cloning vectors, expression vectors, shuttle vectors, plasmids, phage particles, cassettes and the like.

An "expression vector" as used herein means a DNA construct comprising a DNA sequence which is operably linked to a suitable control sequence capable of effecting expression of the DNA in a suitable host. Such control sequences may include a promoter to effect transcription, an optional operator sequence to control transcription, a sequence encoding suitable ribosome binding sites on the mRNA, enhancers and sequences which control termination of transcription and translation. The "expression vector" may be generated recombinantly or synthetically, with a series of specified nucleic acid elements that permit transcription of a particular nucleic acid in a target cell.

The term "selective marker" refers to a gene capable of expression in a host that allows for ease of selection of those hosts containing an introduced nucleic acid or vector. Examples of selectable markers include but are not limited to antimicrobials (*e*.*g*., hygromycin, bleomycin, or chloramphenicol) and/or genes that confer a metabolic advantage, such as a nutritional advantage on the host cell.

"Host strain" or "host cell" means a suitable host for an expression vector or DNA construct comprising a polynucleotide encoding a cellulase according to the invention. Specifically, host strains may be bacterial such as *Streptomyces sp.* or *Bacillus sp.* In an embodiment of the invention, "host cell" means both the cells and protoplasts created from the cells of a filamentous fungal strain and particularly a *Trichoderma sp.* or an *Aspergillus sp*.

### Preferred Embodiments

### A. Host Organisms

Host cells that may be used according to the invention include both bacterial and fungal cells. Preferred fungal host cells include filamentous fungal cells such as *Aspergillus* and *Trichoderma* cells. Preferred bacterial host cells include *Bacillus, Mycobacterium, Actinomyces* and *Streptomyces* cells. Particularly preferred host cells include *E. coli, B. subtilis, B. licheniformis, B. lentus, B. brevis, B. stearothermophilus, B. alkalophilus, B. amyloliquefaciens, B. coagulans, B. circulans, B. lautus, B. megatherium,* and *B. thuringiensis.* Particularly preferred host cells also include *Streptomyces* such as *S. griseus, S. lividans, S. rubiginosis, S. natalensis, S. coelicolor* and *S. avermitilis.* In a particularly preferred embodiment, the host cell is a strain of *S. lividans* and most particularly strains TK23 and/or TK24.

In one embodiment the host cells have been modified so that the production of geosmin or geosmin-like compounds is reduced or eliminated. Geosmin is a chemical produced by a common bacterium, *Streptomyces sp.* It is a greek word and translates as "smell of the earth". Thus, the reduction or elimination of geosmin or geosmin-like compounds will result in a less smelly composition.

### B. DNA constructs and Vectors

The nucleic acid construct of the invention comprising a sequence encoding the novel cellulase may be prepared synthetically by established standard methods, e.g., the phosphoramidite method described by Beaucage and Caruthers, (1981) Tetrahedron Letters 22:1859-1869, or the method described by Matthes et al., (1984) EMBO Journal 3: 801-805. The nucleic acid construct may be of mixed synthetic and genomic origin and may be prepared by ligating fragments of synthetic or genomic DNA. The nucleic acid construct may also be prepared by polymerase chain reaction using specific primers, for instance as described in U.S. Pat. No. 4,683,202 or Saiki et al., Science 239 (1988), 487-491.

A DNA construct of the invention may be inserted into a vector, such as an expression vector. A variety of vectors suitable for the cloning, transformation and expression of polypeptides in fungus, yeast and bacteria are known by those of skill in the art. Typically, the vector or cassette will contain a promoter of the invention, optionally a signal sequence, a coding region of interest and a terminator sequence. In preferred embodiments, the vector will include one or more cloning sties located between the signal sequence and the terminator sequences.

In some preferred embodiments, when a cellulase gene is transferred into a *Streptomyces* host cell, transformation includes use of a vector including a promoter of the invention, a nucleic acid coding for a signal sequence derived from a *Streptomyces* cellulase gene, preferably a *Streptomyces lividans* cellulase gene, and a polynucleotide encoding a bacterial cellulase, particularly a cellulase gene derived from a *Streptomyces* strain, most particularly a *S. lividans* cellulase gene. The signal sequence may also be derived from other signal sequences of a *Streptomyces* strain, and in particularly *S. lividans.*

Exemplary vectors that may be used in the practice of the invention include pSEGCT pSEGCT11 AG8, and pSEACT. Construction of such vectors is well known in the art and reference is made to U. S. Pat. No. 6,287,839; U.S. Pat. No. 6,562,612 and International Publication No. WO 02/50245. Construction of pSEGCT involves the use of two plasmids pIJ486, which is described in Ward et al., (1986) Mol. Gen. Genet. 203:468 - 478 and pIJ488, which is described in Yanisch-Perron et al., (1985) Gene 33: 103-119. In addition, reference is made to Hopwood et al., (1983) J. Gen. Microbiol. 129:2257 - 2260. Other vectors that may be used include pSEA4CT-11AG8, pKB105 and pKB107 as described in the examples.

### C. Transformation of host cells

A vector of the invention will be transformed into a host cell. General transformation techniques are known in the art (Ausubel et al., 1994, CURRENT PROTOCOLS IN MOLECULAR BIOLOGY and Campbell et al., 1989 Curr. Genet 16:53-56). Some of these general techniques include, but are not limited to the use of a particle or gene gun (biolistics), permeabilization of filamentous fungi cells walls prior to the transformation process (*e.g*., by use of high concentrations of alkali, *e.g*., 0.05 M to 0.4 M CaC1₂ or lithium acetate), protoplast fusion, electroporation, or agrobacterium mediated transformation (US Pat. No. 6,255,115) and the treatment of protoplasts or spheroplasts with polyethylene glycol and CaCl₂ is described in Campbell, et al., (1989) Curr. Genet. 16:53-56, 1989 and Penttila, M. et al., (1988) Gene, 63:11-22.

Transformation and expression methods for bacteria are disclosed in Brigidi, DeRossi, Bertarini, Riccardi and Matteuzzi, (1990), FEMS Microbiol. Lett. 55: 135-138. A preferred general transformation and expression protocol for protease deleted *Bacillus* strains is provided in Ferrari et al., U.S. Pat. No. 5,264,366.

Transformation and expression in *Streptomyces* can be found in Hopwood et al., GENETIC MANIPULATION OF STREPTOMYCES: A LABORATORY MANUAL, (1985) John Innis Foundation, Norwich UK.

In other embodiments, transformation and expression in *Aspergillus* and *Trichoderma* is described in, for example U.S. Pat. No. 5,364,770; U.S. Pat. No. 6,022,725; and Nevalainen et al., 1992, The Molecular Biology of Trichoderma and its Application to the Expression of Both Homologous and Heterologous Genes, in MOLECULAR INDUSTRIAL MYCOLOGY, Eds. Leon and Berka, Marcel Dekker, Inc. pp. 129 - 148.

### D. Cell culture

Host cells and transformed cells can be cultured in conventional nutrient media. The culture media for transformed host cells may be modified as appropriate for activating promoters and selecting transformants. The specific culture conditions, such as temperature, pH and the like, may be those that are used for the host cell selected for expression, and will be apparent to those skilled in the art. In addition, preferred culture conditions may be found in the scientific literature such as Sambrook, (1982) *supra*; Kieser, T, MJ. Bibb, MJ. Buttner, KF Chater, and D.A. Hopwood (2000) PRACTICAL STREPTOMYCES GENETICS. John Innes Foundation, Norwich UK; Harwood, et al., (1990) MOLECULAR BIOLOGICAL METHODS FOR BACILLUS, John Wiley and/or from the American Type Culture Collection (ATCC; www.atcc.org). Stable transformants of fungal host cells, such as *Trichoderma* cells can generally be distinguished from unstable transformants by their faster growth rate or the formation of circular colonies with a smooth rather than ragged outline on solid culture medium.

### E. Recovery of Expressed Polypeptides

A polypeptide produced by the transformed host cell may be recovered from the culture medium by conventional procedures including separating the host cells from the medium by centrifugation or filtration, or if necessary, disrupting the cells and removing the supernatant from the cellular fraction and debris.

### F. Methods for purifying the protein

Typically after clarification, the proteinaecous components of the supernatant or filtrate are precipitated by means of a salt, e.g., ammonium sulphate, The precipitated proteins are then solublized and may be purified by a variety of chromatographic procedures, e.g., ion exchange chromatography, gel filtration chromatography, affinity chromatography; and other art-recognized procedures. In some preferred embodiments, for the production of a cellulase it is preferred to cultivate the host cells under alkaline conditions using media containing a cellulase-based energy source.

### G. Utility

Treatment of textiles according to the present invention contemplates textile processing or cleaning with a composition comprising a cellulase of this invention. Such treating includes, but is not limited to, stonewashing, modifying the texture, feel and/or appearance of cellulose-containing fabrics or other techniques used during manufacturing or cleaning/reconditioning of cellulose-containing fabrics. Additionally, treating within the context of this invention contemplates the removal of "immature" or "dead" cotton from cellulosic fabric or fibers. Immature cotton is significantly more amorphous than mature cotton and because of, for example, uneven dyeing. Fabrics are evaluated subjectively using certain criteria such as the thickness, softness, stiffness, smoothness and fullness of the fabric. Fabric hand refers to a person's subjective assessment of a textile material obtained from the sense of touch, in particular softness, and is evaluated by individuals on a panel resulting in a panel score. Alternatively, hand can be evaluated objectively using the Kawabata evaluation system. See, for example, Kawabata, S., "The Standardization and Analysis of Hand Evaluation," Textile Machinery Society of Japan, Osaka, 1980.

The composition contemplated in the present invention further includes a cellulase component for use in washing a soiled manufactured cellulose-containing fabric. For example, a cellulase of this invention may be used in a detergent composition for washing laundry. Detergent compositions useful in accordance with the present invention include special formulations such as pre-wash, pre-soak and home-use color restoration compositions. Such treating compositions, as described herein, may be in the form of a concentrate which requires dilution or in the form of a dilute solution or form which can be applied directly to the cellulose-containing fabric. General treatment techniques for cellulase treatment of textiles are described in, for example, EP Publication No. 220 016 and GB Application Nos. 1,368,599 and 2,095,275.

Treatment of a cellulosic material according to the present invention further contemplates the treatment of animal feed, pulp and/or paper, food and grain for purposes known in the art. For example, cellulases are known to increase the value of animal feed, improve the drainability of wood pulp, enhance food products and reduce fiber in grain during the grain wet milling process or dry milling process.

Treating waste paper with the cellulase described herein can deink the paper. Thus, the use of the cellulase described herein in the process of manufacturing recycled paper from waste paper can reduce ink-remaining fiber greatly to thereby improve the whiteness of the waste paper. Examples of waste paper which may be used in the invention include used printed paper such as used newspaper, used magazine paper and low-grade or middle-grade used printed paper containing mechanical pulp and chemical pulp; used wood-free paper composed of chemical pulp; and coated paper thereof. The term "deinking agent" used herein means those drugs commonly used in the deinking of waste paper, including alkali such as NaOH or Na₂CO₃ soda silicate, hydrogen peroxide, phosphates, anionic or nonionic surfactants, capturing agents such as oleic acid, pH stabilizers as aids, chelating agents, or dispersants.

Treating paper pulp with the cellulase described herein can significantly improve the freeness (drainage) of the pulp without remarkable reduction in its strength. Thus, the present cellulase provides a method of improving the freeness of paper pulp, comprising a step of treating the paper pulp with a cellulase described herein. Examples of pulp, which can be treated by the method of the invention, include waste paper pulp, recycled board pulp, kraft pulp, sulfite pulp or processed/thermally treated pulp, and other high-yield pulp.

Furthermore, the digestibility of glucans in animal feeds can be improved by using the cellulase described herein in such feeds. Thus, provided herein is a method of improving the digestibility of animal feeds, comprising a step of treating the animal feed with the cellulase described herein.

Treating according to the instant invention comprises preparing an aqueous solution which contains an effective amount of a cellulase or a combination of cellulases together with other optional ingredients including, for example, a buffer, a surfactant, and/or a dispersing agent. An effective amount of a cellulase enzyme composition is a concentration of cellulase enzyme sufficient for its intended purpose. Thus, for example, an "effective amount" of cellulase in a stonewashing composition according to the present invention is that amount which will provide the desired effect, *e.g*., to produce a worn and faded look in seams and on fabric panels. Similarly, an "effective amount" of cellulase in a composition intended for improving the feel and/or appearance of a cellulose-containing fabric is the amount that produces measurable improvements in the feel, *e.g*., improving the smoothness of the fabric, or appearance, *e.g*., removing pills and fibrils which tend to reduce the sharpness in appearance of a fabric. The amount of cellulase employed is also dependent on the equipment employed, the process parameters employed (the temperature of the cellulase treatment solution, the exposure time to the cellulase solution, and the like), the cellulase activity (*e.g*., a particular solution will require a lower concentration of cellulase where a more active cellulase composition is used as compared to a less active cellulase composition) and fabric type. The exact concentration of cellulase in the aqueous treatment solution to which the fabric to be treated is added can be readily determined by the skilled artisan based on the above factors as well as the desired result. In stonewashing processes, it has generally been preferred that the cellulase be present in the aqueous treating solution in a concentration of from about 0.5 to 5,000 ppm and most preferably about 10 to 200 ppm total protein. In compositions for the improvement of feel and/or appearance of a cellulose-containing fabric, it has generally been preferred that the cellulase be present in the aqueous treating solution in a concentration of from about 0.1 to 2000 ppm and most preferably about 0.5 to 200 ppm total protein.

In a preferred treating embodiment, a buffer is employed in the treating composition such that the concentration of buffer is sufficient to maintain the pH of the solution within the range wherein the employed cellulase exhibits activity. The pH at which the cellulase exhibits activity depends on the nature of the cellulase employed. The exact concentration of buffer employed will depend on several factors which the skilled artisan can readily take into account. For example, in a preferred embodiment, the buffer as well as the buffer concentration are selected so as to maintain the pH of the final cellulase solution within the pH range required for optimal cellulase activity. The determination of the optimal pH range of the cellulases of the invention can be ascertained according to well-known techniques. Suitable buffers at pH within the activity range of the cellulase are also well known to those skilled in the art in the field.

In addition to cellulase and a buffer, the treating composition may optionally contain a surfactant. Suitable surfactants include any surfactant compatible with the cellulase being utilized and the fabric including, for example, anionic, non-ionic and ampholytic surfactants. Suitable anionic surfactants include, but are not limited to, linear or branched alkylbenzenesulfonates; alkyl or alkenyl ether sulfates having linear or branched alkyl groups or alkenyl groups; alkyl or alkenyl sulfates; olefinsulfonates; alkanesulfonates and the like. Suitable counter ions for anionic surfactants include, but are not limited to, alkali metal ions such as sodium and potassium; alkaline earth metal ions such as calcium and magnesium; ammonium ion; and alkanolamines having 1 to 3 alkanol groups of carbon number 2 or 3. Ampholytic surfactants include, *e.g*., quaternary ammonium salt sulfonates, and betaine-type ampholytic surfactants. Such ampholytic surfactants have both the positive and negative charged groups in the same molecule. Nonionic surfactants generally comprise polyoxyalkylene ethers, as well as higher fatty acid alkanolamides or alkylene oxide adduct thereof, and fatty acid glycerine monoesters. Mixtures of surfactants can also be employed in manners known to those skilled in the art.

A concentrated cellulase composition can be prepared for use in the methods described herein. Such concentrates contain concentrated amounts of the cellulase composition described above, buffer and surfactant, preferably in an aqueous solution. When so formulated, the cellulase concentrate can readily be diluted with water so as to quickly and accurately prepare cellulase preparations having the requisite concentration of each constituent. When aqueous concentrates are formulated, these concentrates can be diluted so as to arrive at the requisite concentration of the components in the cellulase solution as indicated above. As is readily apparent, such cellulase concentrates permit facile formulation of the cellulase solutions as well as permit feasible transportation of the composition to the location where it will be used. The treating concentrate can be in any art-recognized form, for example, liquid, emulsion, gel, or paste. Such forms are well known to those skilled in the art.

When a solid cellulase concentrate is employed, the cellulase composition may be a granule, a powder, an agglomerate or a solid disk. The granules can be formulated so as to contain materials to reduce the rate of dissolution of the granules into the wash medium. Such materials and granules are disclosed in U.S. Patent No. 5,254,283.

Other materials can also be used with or placed in the cellulase composition of the present invention as desired, including stones, pumice, fillers, solvents, enzyme activators, and anti-redeposition agents depending on the eventual use of the composition.

By way of example, stonewashing, methods will be described in detail, however, the parameters described are readily modified by the skilled artisan for other applications, *i.e*., improving the feel and/or appearance of a fabric. The cellulose-containing fabric is contacted with the cellulase containing stonewashing composition containing an effective amount of the cellulase by intermingling the treating composition with the stonewashing composition, and thus bringing the cellulase enzyme into proximity with the fabric. Subsequently, the aqueous solution containing the cellulase and the fabric is agitated. If the treating composition is an aqueous solution, the fabric may be directly soaked in the solution. Similarly, where the stone washing composition is a concentrate, the concentrate is diluted into a water bath with the cellulose-containing fabric. When the stonewashing composition is in a solid form, for example a pre-wash gel or solid stick, the stonewashing composition may be contacted by directly applying the composition to the fabric or to the wash liquor.

The cellulose-containing fabric is incubated with the stonewashing solution under conditions effective to allow the enzymatic action to confer a stonewashed appearance to the cellulose-containing fabric. For example, during stonewashing, the pH, liquor ratio, temperature and reaction time may be adjusted to optimize the conditions under which the stonewashing composition acts. "Effective conditions" necessarily refers to the pH, liquor ratio, and temperature which allow the cellulase enzyme to react efficiently with cellulose-containing fabric, in this case to produce the stonewashed effect. It is within the skill of those in the art to maximize conditions for using the stonewashing compositions according to the present invention.

The liquor ratios during stonewashing, *i*.*e*., the ratio of weight of stonewashing composition solution (*i*.*e*., the wash liquor) to the weight of fabric, employed herein is generally an amount sufficient to achieve the desired stonewashing effect in the denim fabric and is dependent upon the process used. Preferably, the liquor ratios are from about 3:1 to about 50:1; more preferably from about 5:1 to about 20:1, and most preferably from about 10:1 to about 15:1.

Reaction temperatures during stonewashing with the present stonewashing compositions are governed by two competing factors. Firstly, higher temperatures generally correspond to enhanced reaction kinetics, *i*.*e*., faster reactions, which permit reduced reaction times as compared to reaction times required at lower temperatures. Accordingly, reaction temperatures are generally at least about 10°C and greater. Secondly, cellulase is a protein which loses activity beyond a given reaction temperature, which temperature is dependent on the nature of the cellulase used. Thus, if the reaction temperature is permitted to go too high, the cellulolytic activity is lost as a result of the denaturing of the cellulase. While standard temperatures for cellulase usage in the art are generally in the range of 35°C to 65°C, and these conditions would also be expected to be suitable for the cellulase of the invention, the optimal temperature conditions should be ascertained according to well known techniques with respect to the specific cellulase used.

Reaction times are dependent on the specific conditions under which the stonewashing occurs. For example, pH, temperature and concentration of cellulase will all affect the optimal reaction time. Generally, reaction times are from about 5 minutes to about 5 hours, and preferably from about 10 minutes to about 3 hours and, more preferably, from about 20 minutes to about 1 hour.

According to yet another preferred embodiment of the present invention, the cellulase of the invention may be employed in a detergent composition. The detergent compositions according to the present invention are useful as pre-wash compositions, pre-soak compositions, or for cleaning during the regular wash or rinse cycle. Preferably, the detergent composition of the present invention comprises an effective amount of cellulase, a surfactant, and optionally includes other ingredients described below.

An effective amount of cellulase employed in the detergent compositions of this invention is an amount sufficient to impart the desirable effects known to be produced by cellulase on cellulose-containing fabrics, for example, depilling, softening, anti-pilling, surface fiber removal, anti-graying and cleaning. Preferably, the cellulase in the detergent composition is employed in a concentration of from about 10 ppm to about 20,000 ppm of detergent..

The concentration of cellulase enzyme employed in the detergent composition is preferably selected so that upon dilution into a wash medium, the concentration of cellulase enzyme is in a range of about 0.01 to about 1000 ppm, preferably from about 0.02 ppm to about 500 ppm, and most preferably from about 0.5 ppm to about 250 ppm total protein. The amount of cellulase enzyme employed in the detergent composition will depend on the extent to which the detergent will be diluted upon addition to water so as to form a wash solution.

The detergent compositions of the present invention may be in any art recognized form, for example, as a liquid, in granules, in emulsions, in gels, or in pastes. Such forms are well known to the skilled artisan. When a solid detergent composition is employed, the cellulase is preferably formulated as granules. Preferably, the granules can be formulated so as to additionally contain a cellulase protecting agent. The granule can be formulated so as to contain materials to reduce the rate of dissolution of the granule into the wash medium. Such materials and granules are disclosed in U.S. Patent No. 5,254,283.

The detergent compositions of this invention employ a surface active agent, *i*.*e*., surfactant, including anionic, non-ionic and ampholytic surfactants well known for their use in detergent compositions.

Suitable anionic surfactants for use in the detergent composition of this invention include linear or branched alkylbenzenesulfonates; alkyl or alkenyl ether sulfates having linear or branched alkyl groups or alkenyl groups; alkyl or alkenyl sulfates; olefinsulfonates; and alkanesul-fonates. Suitable counter ions for anionic surfactants include alkali metal ions such as sodium and potassium; alkaline earth metal ions such as calcium and magnesium; ammonium ion; and alkanolamines having 1 to 3 alkanol groups of carbon number 2 or 3. Ampholytic surfactants include quaternary ammonium salt sulfonates, and betaine-type ampholytic surfactants. Such ampholytic surfactants have both the positive and negative charged groups in the same molecule. Nonionic surfactants generally comprise polyoxyal-kylene ethers, as well as higher fatty acid alkanolamides or alkylene oxide adduct thereof, fatty acid glycerine monoesters, and the like. Suitable surfactants for use in this invention are disclosed in British Patent Application No. 2 094 826 A, the disclosure of which is incorporated herein by reference. Mixtures of such surfactants can also be used. The surfactant or a mixture of surfactants is generally employed in the detergent compositions of this invention in an amount from about 1 weight percent to about 95 weight percent of the total detergent composition and preferably from about 5 weight percent to about 45 weight percent of the total detergent composition. In addition to the cellulase composition and the surfactant(s), the detergent compositions of this invention can optionally contain one or more of the following components:

### Hydrolases Except Cellulase

Suitable hydrolases include carboxylate ester hydrolase, thioester hydrolase, phosphate monoester hydrolase, and phosphate diester hydrolase which act on the ester bond; glycoside hydrolase which acts on glycosyl compounds; an enzyme that hydrolyzes N-glycosyl compounds; thioether hydrolase which acts on the ether bond; and a-aminoacyl-peptide hydrolase, peptidyl-amino acid hydrolase, acyl-amino acid hydrolase, dipeptide hydrolase, and peptidyl-peptide hydrolase which act on the peptide bond. Preferable among them are carboxylate ester hydrolase, glycoside hydrolase, and peptidyl-peptide hydrolase. Suitable hydrolases include (1) proteases belonging to peptidyl-peptide hydrolase such as pepsin, pepsin B, rennin, trypsin, chymotrypsin A, chymotrypsin B, elastase, enterokinase, cathepsin C, papain, chymopapain, ficin, thrombin, fibrinolysin, renin, subtilisin, aspergillopeptidase A, collagenase, clostridiopeptidase B, kallikrein, gastrisin, cathepsin D., bromelin, keratinase, chymotrypsin C, pepsin C, aspergillopeptidase B, urokinase, carboxypeptidase A and B, and aminopeptidase; (2) glycoside hydrolases (cellulase which is an essential ingredient is excluded from this group) α-amylase, β-amylase, gluco amylase, invertase, lysozyme, pectinase, chitinase, and dextranase. Preferably among them are α-amylase and β-amylase. They function in acid to neutral systems, but one which is obtained from bacteria exhibits high activity in an alkaline system; (3) carboxylate ester hydrolase including carboxyl esterase, lipase, pectin esterase, and chlorophyllase. Especially effective among them is lipase.

The hydrolase other than cellulase is incorporated into the detergent composition as much as required according to the purpose. It should preferably be incorporated in an amount of 0.001 to 5 weight percent, and more preferably 0.02 to 3 weight percent, in terms of purified protein. This enzyme should be used in the form of granules made of crude enzyme alone or in combination with other components in the detergent composition: Granules of crude enzyme are used in such an amount that the purified enzyme is 0.001 to 50 weight percent in the granules. The granules are used in an amount of 0.002 to 20 and preferably 0.1 to 10 weight percent. As with cellulases, these granules can be formulated so as to contain an enzyme protecting agent and a dissolution retardant material.

### Cationic Surfactants and Long-Chain Fatty Acid Salts

Such cationic surfactants and long-chain fatty acid salts include saturated or unsaturated fatty acid salts, alkyl or alkenyl ether carboxylic acid salts, α-sulfofatty acid salts or esters, amino acid-type surfactants, phosphate ester surfactants, quaternary ammonium salts including those having 3 to 4 alkyl substituents and up to 1 phenyl substituted alkyl substituents. Suitable cationic surfactants and long-chain fatty acid salts are disclosed in British Patent Application No. 2 094 826 A. The composition may contain from about 1 to about 20 weight percent of such cationic surfactants and long-chain fatty acid salts.

### Builders

### A. Divalent sequestering agents

The composition may contain from about 0 to about 50 weight percent of one or more builder components selected from the group consisting of alkali metal salts and alkanolamine salts of the following compounds: phosphates, phosphonates, phosphonocarboxylates, salts of amino acids, aminopolyacetates high molecular electrolytes, non-dissociating polymers, salts of dicarboxylic acids, and aluminosilicate salts. Suitable divalent sequestering gents are disclosed in British Patent Application No. 2 094 826 A.

### B. Alkalis or inorganic electrolytes

The composition may contain from about 1 to about 50 weight percent, preferably from about 5 to about 30 weight percent, based on the composition of one or more alkali metal salts of the following compounds as the alkalis or inorganic electrolytes: silicates, carbonates and sulfates as well as organic alkalis such as triethanolamine, diethanolamine, monoethanolamine and triisopropanolamine.

### Antiredeposition Agents

The composition may contain from about 0.1 to about 5 weight percent of one or more of the following compounds as antiredeposition agents: polyethylene glycol, polyvinyl alcohol, polyvinylpyrrolidone and carboxymethylcellulose.

Among them, a combination of carboxymethyl-cellulose and/or polyethylene glycol with the cellulase composition of the present invention provides for an especially useful dirt removing composition.

### Bleaching Agents

The use of the cellulase of the present invention in combination with a bleaching agent such as potassium monopersulfate, sodium percarbonate, sodium perborate, sodium sulfate/hydrogen peroxide adduct and sodium chloride/hydrogen peroxide adduct or/and a photo-sensitive bleaching dye such as zinc or aluminum salt of sulfonated phthalocyanine further improves the detergenting effects. Similarly, bleaching agents and bleach catalysts as described in EP 684 304 may be used.

### Bluing Agents and Fluorescent Dyes

Various bluing agents and fluorescent dyes may be incorporated in the composition, if necessary. Suitable bluing agents and fluorescent dyes are disclosed in British Patent Application No. 2 094 826 A.

### Caking Inhibitors

The following caking inhibitors may be incorporated in the powdery detergent: p-toluehesulfonic acid salts, xylenesulfonic acid salts, acetic acid salts, sulfosuccinic acid salts, talc, finely pulverized silica, amorphous silicas, clay, calcium silicate (such as MicroCell of Johns Manville Co.), calcium carbonate and magnesium oxide.

### Antioxidants

The antioxidants include, for example, tert-butyl-hydroxytoluene, 4,4'-butylidenebis(6-tert-butyl-3-methylphenol), 2,2'-butylidenebis(6-tert-butyl-4-methylphenol), monostyrenated cresol, distyrenated cresol, monostyrenated phenol, distyrenated phenol and 1,1-bis(4-hydroxy-phenyl)cyclohexane.

### Solubilizers

The solubilizers include, for example, lower alcohols such as ethanol, benzenesulfonate salts, lower alkylbenzenesulfonate salts such as p-toluenesulfonate salts, glycols such as propylene glycol, acetylbenxene-sulfonate salts, acetamides, pyridinedicarboxylic acid amides, benzoate salts and urea.

The detergent composition of the present invention can be used in a broad pH range from acidic to alkaline pH. In a preferred embodiment, the detergent composition of the present invention can be used in mildly acidic, neutral or alkaline detergent wash media having a pH of from above 5 to no more than abut 12.

Aside from the above ingredients, perfumes, buffers, preservatives, dyes and the like can be used, if desired, with the detergent compositions of this invention. Such components are conventionally employed in amounts heretofore used in the art.

When a detergent base used in the present invention is in the form of a powder, it may be one which is prepared by any known preparation methods including a spray-drying method and a granulation method. The detergent base obtained particularly by the spray-drying method, agglomeration method, dry mixing method or non-tower route methods are preferred. The detergent base obtained by the spray-drying method is not restricted with respect to preparation conditions. The detergent base obtained by the spray-drying method is hollow granules which are obtained by spraying an aqueous slurry of heat-resistant ingredients, such as surface active agents and builders, into a hot space. After the spray-drying, perfumes, enzymes, bleaching agents, inorganic alkaline builders may be added. With a highly dense, granular detergent base obtained such as by the spray-drying-granulation or agglomeration method, various ingredients may also be added after the preparation of the base.

When the detergent base is a liquid, it may be either a homogeneous solution or a nonhomogeneous dispersion. For removing the decomposition of carboxymethylcellulose by the cellulase in the detergent, it is desirable that carboxymethylcellulose is granulated or coated before the incorporation in the composition.

The detergent compositions of this invention may be incubated with cellulose-containing fabric, for example soiled fabrics, in industrial and household uses at temperatures, reaction times and liquor ratios conventionally employed in these environments.

Detergents according to the present invention may additionally be formulated as a pre-wash in the appropriate solution at an intermediate pH where sufficient activity exists to provide desired improvements softening, depilling, pilling prevention, surface fiber removal or cleaning. When the detergent composition is a pre-soak (*e.g*., pre-wash or pre-treatment) composition, either as a liquid, spray, gel or paste composition, the cellulase enzyme is generally employed from about 0.0001 to about 1 weight percent based on the total weight of the pre-soak or pre-treatment composition. In such compositions, a surfactant may optionally be employed and when employed, is generally present at a concentration of from about 0.005 to about 20 weight percent based on the total weight of the pre-soak. The remainder of the composition comprises conventional components used in the pre-soak, *i*.*e*., diluent, buffers, other enzymes (proteases), and the like at their conventional concentrations.

It is contemplated that compositions comprising cellulase enzymes described herein can be used in home use as a stand alone composition suitable for restoring color to faded fabrics (see, for example, U.S. Patent No. 4,738,682) as well as used in a spot-remover and for depilling and antipilling (pilling prevention).

The use of the cellulase according to the invention may be particularly effective in feed additives and in the processing of pulp and paper. These additional industrial applications are described in, for example, PCT Publication No. 95/16360 and Finnish Granted Patent No. 87372, respectively.

In order to further illustrate the present invention and advantages thereof, the following specific examples are given with the understanding that they are being offered to illustrate the present invention and should not be construed in any way as limiting its scope.

In the experimental disclosure which follows, the following abbreviations apply: eq (equivalents); M (Molar); µM (micromolar); N (Normal); mol (moles); mmol (millimoles); µmol (micromoles); nmol (nanomoles); g (grams); mg (milligrams); kg (kilograms); µg (micrograms); L (litters); ml (milliliters); µl (microliters); cm (centimeters); mm (millimeters); µm (micrometers); nm (nanometers); °C. (degrees Centigrade); h (hours); min (minutes); sec (seconds); msec (milliseconds); Cl (Curies) mCi (milliCuries); µCi (microCuries); TLC (thin layer achromatography); Ts (tosyl); Bn (benryl); Ph (phenyl); Ms (mesyl); Et (ethyl), Me (methyl).

### EXAMPLES

The present invention is described in further detain in the following examples which are not in any way intended to limit the scope of the invention as claimed. The attached Figures are meant to be considered as integral parts of the specification and description of the invention. The following examples are offered to illustrate, but not to limit the claimed invention.

### Example 1

### Construction of vectors

This example illustrates the construction of plasmids comprising the novel cellulase catalytic core.

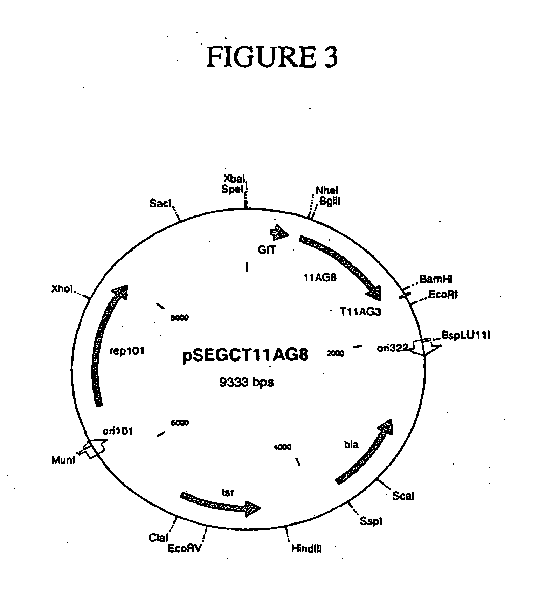

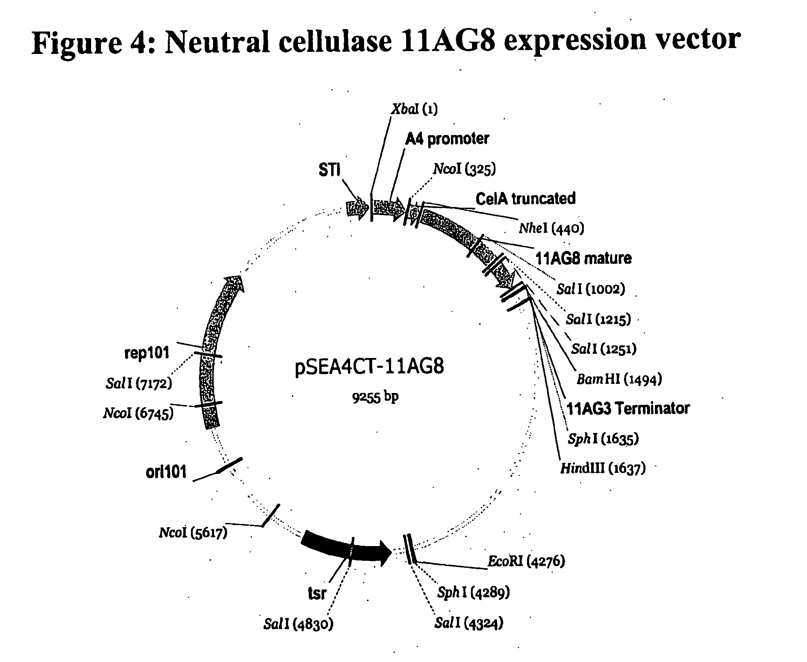

A pSEGCT11AG8 vector containing a GI promoter as shown herein as Figure 3 and described in example 6 of U.S. Pat. No. 6,562,612 was used as the basis for the production of the vectors used in the present invention. The pSEA4CT-11AG8 vector construction is described in Example 2 of United States Patent Application Serial Number 10/992,149 (filed November 18, 2004) and reference is made to Figure 4 of the present application (map of pSEA4CT-11 AG8).

Plasmid pKB105 was constructed from plasmid pSEA4CT-11 AG8 by replacing the segment encoding the full-length 11 AG8 cellulase with a sequence encoding the novel cellulase catalytic core. See Figure 5 (wherein the sequence encoding the novel cellulase catalytic core is designated as "11AG8 Core I". Figure 6 shows the pKB105 vector.

The novel cellulase catalytic core expression vector, pKB107, was derived from pKB105. Removal of the E.coli DNA sequences in pKB105 produced the plasmid pKB107. To remove the E. coli sequences pKB105was digested with Sphl, EcoRI and HindIII overnight digestion at 37°C. The digested DNA was purified using a Qiagen kit and then re-ligated for transformation of *Streptomyces* host cells. Figure 7 shows the pKB107 vector.

### Example 2

### Expression and Activity

The following example describes the expression and activity of the novel cellulase.

### 2A. TRANSFORMATION AND EXPRESSION

The expression vectors, pSEA4CT-11AG8 and pKB107, constructed in Example 1 were used in this example.

In these experiments, the host *Streptomyces lividans* cells were transformed with the vectors described above. The transformation techniques were the protoplast method described in Hopwood, et al., GENETIC MANIPULATION OF STREPTOMYCES, A LABORATORY MANUAL. The John Innes Foundation, Norwich, United Kingdom (1985).

*Streptomyces lividans* cells were transformed with one of the expression vectors as described above. Transformed cells were plated on azo-CMC plates and colonies expressing a cellulase were identified by production of a "halo". Colonies producing a halo were grown in TS in shake flasks for 3 days in the presence of 50 ug/ml thiostrepton at 30°C. Cells were then transferred to a production medium free of antibiotics and growth was continued for another three days. Samples were taken for enzyme activity assay.

TS = 16 g Difco tryptone, 4 g Difco soytone, 20 g caseine (hydolysate) sigm and 5g K₂HPO₄ brought to 1 liter. After autoclaving 50% filtered sterilized glucose was added to a final concentration of 1.5%.

Production Media: 2.4 g Citric Acid*H₂O; 8.3 g Biospringer Yeast Extract; 2.4 g (NH₄)₂SO₄; 72.4 g MgSO₄*7H₂O; 0.1 g CaCl₂*2H₂O; 0.3 ml Mazu DF204 (antifoam); 5 ml Streptomyces modified trace elements (1 liter stock solution contains: 250 g Citric acid * H₂O; 3.25 g FeSO₄*7H₂O; 5 g ZnSO₄*7H₂O; 5 g MnSO₄* H₂O; 0.25 g H₃BO₃); 10 g glucose, adjust volume to 1 liter. Adjust pH to 6.9 with NaOH.

### 2B. RECOVERY

One ml of sample was taken from each shake flask and centrifuged at 14,000 rpm. Part of the supernatant was used for the enzyme assay.

Additionally fermentation cultures were run with the transformed cultures. At various time points, samples of the fermentation broths were removed for analysis. Cell matter was removed by centrifugation.

### 2C. MODIFIED ASSAY FOR CELLULASE ACTIVITY

This example demonstrate that a simple, direct, reliable and time-saving assay can be used to evaluate cellulase activity using microtiter plates.

A standard enzyme reference from a previously quantified fermentation sample having an activity of 2201 Unit/mL was used. Dilution buffer: 100mM Sodium Phosphate, pH 8.0 and 0.2um filter sterilized was used to dilute the samples and substrate. The dilution buffer was prepared by mixing 12g NaH₂PO₄ in 800mfl de-ionized water and pH adjust up to pH 8.0 with 6N NaOH was brought to 1.0L final volume and then 0.2um filter sterilized.

The standard enzyme reference was diluted 40x and then 2x serial dilutions three to five times. Shake flask samples were serially diluted 2x, three to five times. Fermentor samples were diluted 20 to 50x, and subsequently serially diluted 2x, three to five times. Thus, there were a total of 3 to 5 samples with different protein concentrations for each experimental sample and the standard enzyme reference.

For the assay, 180 uL of 0.5 mg/ml 2-Nitrophenyl β-D-cellobioside (Sigma) in dilution buffer (see above) was mixed with 20 ul sample in a 96-well plate (Model "9017", Costar, Cambridge, MA) at room temperature.

Cellulase activities were measured by reading absorbance at 405nm using a Spectra MAX250 spectrophotometer (Spectra, Sunnyvale, CA, U.S.A.) for 8 minutes with interval 9 seconds and the kinetic average determined.

The results are shown in Figure 8. The Y-axis is the NPC units/ml. As can be seen removal of the CBD increases neutral cellulase activity dramatically compared to the intact cellulase.

### Example 3

### Wash Performance

The following example compares the wash performance of a granulated experimental novel catalytic core sample KB107C blend (95% KB107C + 5% IndiAge^{®} Neutra L) against a commercial 11AG8 product, IndiAge^{®} Neutra G (Genencor Intl.). The enzymes were dosed using same total ONPC activity per run.

The experimental procedure for the 35 kg denim substrate can be summarized as follows:
Step 1: Desizing (55°C/20 min)
Step 2: Drop & Rinse
Step 3: Cellulase Treatment (55°C/pH 6.5/ 60 min)
Step 4: Cold Rinse (1-2 min)
Step 5: Hot Rinse (70°C/ 5 min)
Step 6: Cold Rinse (1-2 min)
Step 7: Extract at Extractor
Step 8: Drying at tumbling dryer
Step 9: Evaluation

### Trials

Desizing was done with 0.57 g/L formulated Optisize and 0.25g/L Triton X-100 at 55°C for 20 minutes.

Desized denim substrates were treated with new granule (95% KB107C + 5% IndiAge^{®} Neutra L) and IndiAge^{®} Neutra G in a production scale belly washer under the following conditions:
■ Liquor ratio (LR): 15 to 1 (525 L water, 35 Kg desized denim jeans)
■ Surfactant: Lutensol AT80 @ 0.14 g/L
■ Treatment temperature: 55°C pH: 6.5 ± 0.2 (monosodium phosphate buffer with acetic acid pH adjustment))
■ Treatment time: 60 minutes
■ 3 rinses

Various trials were run using the following enzyme dosages: 1) IndiAge^{®} Neutra G @ 1,702 X 10³ units/run; 2) 0.65X granule @ 1,106 X 10³ units/run (65% activity of #1); 3) IndiAge^{®} Neutra G @ 1,702 X 10³ units/run-Duplicate #1; 4) 0.65X granule @ 1106 X 10³ units/run (65% activity of Trial 3)-Duplicate #2; and 5) 0.65X granule @ 1,702 X 10³ units/run.

Five denim legs were randomly selected after cellulose treatment from each run from trials 1-5, and lightly bleached to remove backstaining on the front side of the denim. Bleaching of all the selected denim legs was done together with 10g/L sodium hypochlorite (6.15 % active) and soda ash at 65°C for 20 minutes in the Unimac followed by surfactant wash.

### Evaluation of backstaining and abrasion:

To quantify the backstaining and abrasion levels after the cellulase treatment, 6 reflectometer readings were taken from each denim leg using a Chroma Meter CR-200 by Minolta. CIE L* values were used to quantify the abrasion, and CIE b* values were used to quantify the backstaining. L* indicates lightness and -b* indicates blueness in CIELAB color space. Thus, higher L* indicates higher abrasion and higher -b* indicates higher backstaning.

Measurements were done as described in "Precise Color Communication", by Minolta Camera Co., Ltd, 1993, 2. Hunter, R.S. abd G+Harold, R. " The measurement of Appearance", J. Wiley and Sons, NY, 2nd edition, 1987).

### Results

The trial results demonstrate that the wash strength of the 1X activity commercial IndiAge^{®} Neutra G is about equal to the 0.65X activity of the new Neutra G (95% KB107C+5% IndiAge^{®} Neutra L). The results are present in the Table 1. below, and in Figures 9 and 10.

**Table1: Abrasion and backstaining performances of IndiAge^{®} Neutra G, and 0.65X new Neutra G.**

| **Trial No.** | **Enzymes** | **Dose** | **Abrasion (CIE L*)** | | | **Backstaining (CIE lb*I)** | | |
|---|---|---|---|---|---|---|---|---|
| | | (NPCU/run) | SB/Indigo | Indigo | SB/I-BL | SB/Indigo | Indigo | SB/I-BL |
| **1** | IndiAge^{®} Neutra G | 1702 X 10³ | 30.82 | 22.73 | n/a | 3.88 | 3.40 | n/a |
| **2** | Neutra G-New | 1106 X 10³ | 30.18 | 22.40 | n/a | 3.08 | 2.71 | n/a |
| **3** | IndiAge^{®} Neutra G (DP T1) | 1702 X 10³ | 31.21 | 23.42 | 37.48 | 4.02 | 3.92 | 3.89 |
| **4** | Neutra G-New (DP T2) | 1106 X 10³ | 31.12 | 23.45 | 37.35 | 3.75 | 3.63 | 3.90 |
| **5** | Neutra G-New | 1702 X 10³ | 32.73 | 24.25 | 39.14 | 4.43 | 4.26 | 3.93 |

| | | | | | | | | |
|---|---|---|---|---|---|---|---|---|
| Notes: *SB/Indigo: sulfur bottom/indigo dyed denim with cellulase treatment **Indigo: indigo dyed denim with cellulase treatment ***SB/I-BL: sulfur bottom/indigo dyed denim with cellulase treatment followed by bleaching | | | | | | | | |

Under the conditions tested, the new Neutra G (95% KB107C + 5% IndiAge^{®} Neutra L) at 65% activity showed very similar abrasion performance to IndiAge^{®} Neutra G at 100% activity and the new Neutra G at 100% activity exhibited significantly higher abrasion performance to IndiAge^{®} Neutra G at 100% activity. No significant differences in backstaining performances were observed between the new Neutra G and the IndiAge^{®} Neutra G under similar abrasion performances.

### Example 4

### Differential Scanning Calorimetry

The following example describes the determination of thermostability of the novel cellulase.

KB107C was prepared as described in Example 2 above. The KB107C was then formulated as follows:

| | | |
|---|---|---|
| Sucrose | | 40.0% |
| Sodium citrate, dihydrate | | 2.84% (2.50% anhyd.) |
| Sodium phosphate, dibasic heptahydrate | | 4.72% (2.50% anhyd.) |
| Potassium sorbate | | 0.25% |
| Methyl paraben | | 0.03% |
| Propyl paraben | | 0.01 % |
| Total | | 47.85% |
| | | |
| Activity | | 7500 - 9000 U/g |
| pH | 5.8 - 6.2 | |

Parabens were added as a stock solution: 15% methyl; 5% propyl paraben; 80% propylene glycol. The above formulation is the same as the commercial product, IndiAge^{®} Neutra L.

IndiAge^{®} Neutra L and KB107C were precipitated from the formulation using 1.2 M ammonium sulfate. The precipitate was resuspended and dialyzed into 20 mM sodium phosphate, pH 6.8. A Slide-A-Lyzer 7K dialysis cassette (Pierce, IL) was used for the dialysis and the dialysis was performed over a 24-hour period with four buffer exchanges. The final dialyzate was used to dilute the protein samples. The excessive heat capacity of the samples was measured relative to the final dialyzate as a reference.

Excessive heat capacity curves were measured using an ultrasensitive scanning microcalorimeter VP-DSC E-2000 (Microcal, Inc., Northhampton, Ma.). A heating rate of 90 °C min⁻¹ was applied and the protein concentration was in the range of 0.2 mg/ml. The standard procedure for DSC measurements and the theory of the technique is previously published (Freire, E. (1995) Differential Scanning Calorimetry Methods. Mol. Biol. 41, 191-218).

The thermal stability of IndiAge^{®}Neutra L and KB107C as a function of temperature (20 - 100 °C) indicated a thermal midpoint (Tm) for IndiAge^{®} Neutra L and KB107C of 67.8 °C and 68.7 °C, respectively (see Figure 11). The higher transition temperature of 1°C for KB107C is significant given the sensitivity of this instrumentation. This suggests that the thermodynamic properties of KB107C differ from those of IndiAge® Neutra L, and is consistent with its enhanced performance in application studies.

It is understood that the examples and embodiments described herein are for illustrative purposes only and that various modifications or changes in light thereof will be suggested to persons skilled in the art.

## Claims

1. An isolated cellulase having an amino acid sequence consisting of the amino acid sequence as set out in Figure 1 E.

2. The cellulase according to claim 1, wherein said cellulase is encoded by a DNA sequence as set out in Figure 2.

3. An isolated DNA sequence having the nucleic acid sequence set out in Figure 2.

4. An expression vector comprising the DNA molecule according to claim 3.

5. The expression vector according to claim 4, wherein the expression vector further comprises an aprE promoter or a glaA promoter or an A4 promoter operably linked to the DNA molecule.

6. The expression vector according to claim 5, wherein the expression vector comprises the A4 promoter operably linked to the DNA molecule.

7. A host cell transformed with the expression vector according to any one of claims 4 to 6.

8. The host cell of claim 7, wherein the host cell is a Streptomyces spp or a Bacillus spp; or the host cell is a *Streptomyces spp* in which the cyc2 gene (geosmin pathway gene) has been deleted; or the host cell is a filamentous fungus such as *Aspergillus* or *Trichoderma* or a yeast such as *Saccharomyces.*

9. A composition comprising a cellulase according to claim 1 or claim 2.

10. The composition according to claim 9, where the composition is a detergent composition.

11. A detergent composition comprising a cellulase according to claim 1 or claim 2.

12. A method of producing an enzyme having cellulase activity, comprising:
(a) stably transforming an isolated host cell with an expression vector comprising a polynucleotide as defined in any one of claims 4 to 6;
(b) cultivating said transformed host cell under conditions suitable for said host cell to produce said enzyme; and
(c) recovering said enzyme.

13. A method of producing a cellulase according to claim 1 or claim 2, the method comprising:
(a) growing a transformed host cell according to claim 12 under conditions suitable for expression of said DNA encoding said cellulase; and
(b) collecting the resulting aqueous mixture comprising said cellulase.

14. The method according to claim 13, wherein said cellulase is further purified from said aqueous mixture.

15. The method according to claim 13 or claim 14, wherein said host cell is a Bacillus spp or a Streptomyces spp.

16. A method of treating cellulose-containing fabrics, comprising a step of contacting the cellulose-containing fabrics with the cellulase according to claim 1 or claim 2.

17. The method of claim 16, wherein:
(i) the treatment is stonewashing; or
(ii) the treatment is providing a localized variation in color of colored cellulose-containing fabrics; or
(iii) treatment is providing surface polishing effects on cellulose-containing fabrics; or
(iv) the treatment provides an improved hand feel of cellulose-containing fabrics; or
(v) the treatment provides color clarification on cellulose-containing fabrics; or
(vi) the treatment of the fabrics is performed through soaking, washing or rinsing the fabrics.

18. A method of treating paper pulp, comprising a step of contacting the paper pulp with the cellulase according to claim 1 or claim 2.

19. A method of improving the digestibility of an animal feed, comprising a step of treating a cellulase containing feed with the cellulase according to claim 1 or claim 2

20. The use of the cellulase according to claim 1 or claim 2 as an additive for animal feed, for the treatment of textiles, in the treatment of pulp and paper or in a detergent composition.

## Patentansprüche

1. Isolierte Cellulase mit einer Aminosäuresequenz, die aus der in Figur 1 E dargelegten Aminosäuresequenz besteht.

2. Cellulase nach Anspruch 1, worin für die Cellulase eine wie in Figur 2 dargelegte DNA-Sequenz kodiert.

3. Isolierte DNA-Sequenz mit der in Figur 2 dargelegten Nucleinsäuresequenz.

4. Expressionsvektor, umfassend das DNA-Molekül nach Anspruch 3.

5. Expressionsvektor nach Anspruch 4, worin der Expressionsvektor außerdem einen aprE-Promotor, einen glaA-Promotor oder einen A4-Promotor umfasst, der jeweils an das DNA-Molekül operabel gebunden ist.

6. Expressionsvektor nach Anspruch 5, worin der Expressionsvektor den A4-Promotor, operabel gebunden an das DNA-Molekül, umfasst.

7. Wirtszelle, transformiert mit dem Expressionsvektor nach einem der Ansprüche 4 bis 6.

8. Wirtszelle nach Anspruch 7, worin die Wirtszelle eine Streptomyces-spp. oder eine Bacillus-spp. ist; oder die Wirtszelle eine Streptomyces-spp. ist, in der das cyc2-Gen (Geosmin-Stoffwechselweg-Gen) deletiert wurde; oder die Wirtszelle ein Fadenpilz, wie beispielsweise Aspergillus oder Trichoderma, oder eine Hefe wie Saccharomyces ist.

9. Zusammensetzung, umfassend eine Cellulase nach Anspruch 1 oder Anspruch 2.

10. Zusammensetzung nach Anspruch 9, wobei die Zusammensetzung eine Waschmittelzusammensetzung ist.

11. Waschmittelzusammensetzung, umfassend eine Cellulase nach Anspruch 1 oder Anspruch 2.

12. Verfahren zur Herstellung eines Enzyms mit Cellulaseaktivität, umfassend:
(a) das stabile Transformieren einer isolierten Wirtszelle mit einem ein Polynucleotid umfassenden Expressionsvektor nach einem der Ansprüche 4 bis 6;
(b) das Kultivieren der transformierten Wirtszelle unter Bedingungen, die dazu geeignet sind, dass die Wirtszelle das Enzym produziert; und
(c) das Gewinnen des Enzyms.

13. Verfahren zur Herstellung einer Cellulase nach Anspruch 1 oder Anspruch 2, welches Verfahren Folgendes umfasst:
(a) das Züchten einer transformierten Wirtszelle nach Anspruch 12 unter zur Expression der für die Cellulase kodierenden DNA geeigneten Bedingungen; und
(b) das Sammeln des resultierenden wässrigen Gemischs, das die Cellulase umfasst.

14. Verfahren nach Anspruch 13, worin die Cellulase weiters aus dem wässrigen Gemisch gereinigt wird.

15. Verfahren nach Anspruch 13 oder Anspruch 14, worin es sich bei der Wirtszelle um eine Bacillus-spp. oder eine Streptomyces-spp. handelt.

16. Verfahren zur Behandlung von cellulosehältigen Stoffen, umfassend einen Schritt des Kontaktierens der cellulosehältigen Stoffe mit der Cellulase nach Anspruch 1 oder Anspruch 2.

17. Verfahren nach Anspruch 16, worin:
(i) die Behandlung in Stonewashing besteht; oder
(ii) die Behandlung im Bereitstellen einer lokalisierten Farbvariation farbiger cellulosehältiger Stoffe besteht; oder
(iii) die Behandlung im Bereitstellen von Oberflächenglättungseffekten auf cellulosehältigen Stoffen besteht; oder
(iv) die Behandlung cellulosehältigen Stoffen eine bessere Griffigkeit verleiht; oder
(v) die Behandlung cellulosehältigen Stoffen eine klarere Farbe verleiht; oder
(vi) die Behandlung der Stoffe durch Tränken, Waschen oder Spülen der Stoffe erfolgt.

18. Verfahren zur Behandlung von Papierzellstoff, umfassend den Schritt des Kontaktierens des Papierzellstoffs mit der Cellulase nach Anspruch 1 oder Anspruch 2.

19. Verfahren zur Verbesserung der Verdaulichkeit eines Tierfutters, umfassend den Schritt des Behandelns eines cellulosehaltigen Futters mit der Cellulase nach Anspruch 1 oder Anspruch 2.

20. Verwendung der Cellulase nach Anspruch 1 oder Anspruch 2 als Zusatz für Tierfutter, zur Behandlung von Textilien, bei der Behandlung von Papierzellstoff und Papier oder in einer Waschmittelzusammensetzung.

## Revendications

1. Cellulase isolée ayant une séquence d'acides aminés constituée par la séquence d'acides aminés telle qu'exposée dans la Figure 1E.

2. Cellulase selon la revendication 1, où ladite cellulase est codée par une séquence d'ADN telle qu'exposée dans la Figure 2.

3. Séquence d'ADN isolée ayant la séquence d'acides nucléiques exposée dans la Figure 2.

4. Vecteur d'expression comprenant la molécule d'ADN selon la revendication 3.

5. Vecteur d'expression selon la revendication 4, où le vecteur d'expression comprend en outre un promoteur aprE ou un promoteur glaA ou un promoteur A4 fonctionnellement lié à la molécule d'ADN.

6. Vecteur d'expression selon la revendication 5, où le vecteur d'expression comprend le promoteur A4 fonctionnellement lié à la molécule d'ADN.

7. Cellule hôte transformée par le vecteur d'expression selon l'une quelconque des revendications 4 à 6.

8. Cellule hôte selon la revendication 7, où la cellule hôte est une Streptomyces spp ou une Bacillus spp; ou bien la cellule hôte est une *Streptomyces sppuin* dans laquelle le gène cyc2 (gène de chemin de géosmine) a été effacé; ou bien la cellule hôte est un champignon filamenteux comme *Aspergillus* ou *Trichoderma* ou une levure comme *Saccharomyces.*

9. Composition comprenant une cellulase selon la revendication 1 ou la revendication 2.

10. Composition selon la revendication 9, où la composition est une composition détergente.

11. Composition détergente comprenant une cellulase selon la revendication 1 ou la revendication 2.

12. Procédé de fabrication d'une enzyme ayant une activité de cellulase, comprenant:
(a) transformer d'une manière stable une cellule hôte isolée avec un vecteur d'expression comprenant un polynucléotide tel que défini dans l'une quelconque des revendications 4 à 6;
(b) cultiver ladite cellule hôte transformée sous des conditions qui conviennent pour que ladite cellule hôte produise ladite enzyme; et
(c) récupérer ladite enzyme.

13. Procédé de production d'une cellulase selon la revendication 1 ou la revendication 2, le procédé comprenant:
(a) cultiver une cellule hôte transformée selon la revendication 12 sous des conditions qui conviennent pour l'expression dudit ADN codant pour ladite cellulase; et
(b) recueillir le mélange aqueux qui en résulte comprenant ladite cellulase.

14. Procédé selon la revendication 13, dans lequel ladite cellulase est en outre purifiée dudit mélange aqueux.

15. Procédé selon la revendication 13 ou la revendication 14, dans lequel ladite cellule hôte est une Bacillus spp ou une Streptomyces spp.

16. Procédé de traitement d'étoffes contenant de la cellulose, comprenant une étape consistant à mettre en contact l'étoffe contenant de la cellulose avec la cellulase selon la revendication 1 ou la revendication 2.

17. Procédé selon la revendication 16, dans lequel:
(i) le traitement est le délavage à la pierre; ou
(ii) le traitement est la réalisation d'une variation localisée dans la couleur de l'étoffe colorée contenant de la cellulose; ou
(iii)le traitement entraîne des effets de polissage de surface sur l'étoffe contenant de la cellulose; ou
(iv) le traitement améliore le toucher de l'étoffe contenant de la cellulose; ou
(v) le traitement entraîne une clarification de la couleur sur l'étoffe contenant de la cellulose; ou
(vi) le traitement de l'étoffe est exécuté par trempage, lavage ou rinçage de l'étoffe.

18. Procédé de traitement de la pulpe de papier, comprenant une étape consistant à mettre en contact la pulpe de papier avec la cellulase selon la revendication 1 ou la revendication 2.

19. Procédé d'amélioration de la digestibilité d'aliments pour animaux, comprenant une étape consistant à traiter des aliments contenant de la cellulose avec la cellulase selon la revendication 1 ou la revendication 2.

20. Utilisation de la cellulase selon la revendication 1 ou la revendication 2 comme un additif à des aliments pour animaux, pour le traitement de textiles, dans le traitement de la pulpe de papier ou dans une composition détergente.
